Europäisches Patentamt

European Patent Office

Office européen des brevets

(19)

(11) Numéro de publication: **0 159 230**
**B1**

(12) **FASCICULE DE BREVET EUROPEEN**

(45) Date de publication du fascicule du brevet: **26.10.88**

(21) Numéro de dépôt: **85400501.4**

(51) Int. Cl.⁴: **G 01 N 30/00**

(22) Date de dépôt: **15.03.85**

(54) **Procédé et élément capteur pour détecter la présence dans un fluide en déplacement de certains corps notamment dissous et applications.**

(30) Priorité: **15.03.84 FR 8404021**

(43) Date de publication de la demande:
**23.10.85 Bulletin 85/43**

(45) Mention de la délivrance du brevet:
**26.10.88 Bulletin 88/43**

(84) Etats contractants désignés:
**BE CH DE GB IT LI NL**

(56) Documents cités:
**FR-A-2 187 804**
**FR-A-2 243 965**
**FR-A-2 321 125**
**FR-A-2 327 542**
**FR-A-2 394 084**
**GB-A-1 429 470**

**ANALYTICAL CHEMISTRY, vol. 38, no. 8, juillet 1966, pages 987-996, US; W.J. CAMPBELL et al.: "Micro and trace analysis by a combination of ion exchange resin-loaded papers and x-ray spectrography"**

(73) Titulaire: **Centre Technique Industriel dit: INSTITUT TEXTILE DE FRANCE 35, rue des Abondances B.P. 79 F-92105 Boulogne Billancourt Cedex (FR)**

(72) Inventeur: **Chatelin, Roger Jean 36 Allée des Monts d'Or Domaine de Bois Dieu Lissieu F-69380 Lozanne (FR)**
Inventeur: **Combes, Jean-François Guy Lieuran les Beziers F-34290 Servian (FR)**
Inventeur: **Wattiez, Daniel Léon 4 allée du Bois d'Ars Domaine de Bois Dieu Lissieu F-69380 Lozanne (FR)**
Inventeur: **Boullard, Jacque André 326 Avenue de la Dent de Crolles F-38330 Saint Ismier (FR)**

(74) Mandataire: **Hasenrader, Hubert et al Cabinet BEAU DE LOMENIE 55, rue d'Amsterdam F-75008 Paris (FR)**

Courier Press, Leamington Spa, England.

EP 0 159 230 B1

### Description

L'invention concerne la détection de la présence dans un fluide en déplacement de certains corps notamment dissous.

Elle a trait en particulier à la détection de la pollution dans les fluides naturels, notamment les cours d'eau.

Pour détecter la présence de corps dans un fluide en déplacement, on procède généralement à des prélèvements d'une certaine quantité de fluide sur laquelle sont effectuées différentes analyses permettant de déceler la présence ou l'absence de corps dans le fluide au moment où le prélèvement est réalisé. Ces techniques par prélèvement ne reflètent que l'état du fluide à un moment donné et ne tiennent pas compte des corps qui ont pu être présents entre deux prélèvements. Par ailleurs, elles nécessitent le plus souvent que les analyses soient faites rapidement pour éviter l'évolution du bain telle que par exemple la prolifération bactérienne qui risquerait de perturber les analyses. On procède également à des analyses en continu à l'aide d'appareils placés à demeure dans le fluide, mais dans ce cas il s'agit de l'analyse d'un corps donné, l'appareil en question n'étant capable de détecter la présence ou l'absence que de ce seul corps.

Or on a trouvé, et c'est ce qui fait l'objet de l'invention, un procédé pour détecter la présence, dans un fluide en déplacement, de certains corps notamment dissous, qui donne une indication sur tous les corps en question présents dans le fluide pendant un intervalle de temps donné et qui ne soit pas spécifique d'un corps donné. Les corps, qui sont justiciables du procédé, sont tous les corps présentant une affinité, pour une fonction chimique, suffisante pour que le corps puisse s'associer chimiquement à ladite fonction. La grande majorité des corps concernés sont des corps dissous dans le fluide et pour la clarté de la description nous désignerons tous les corps présentant cette affinité chimique par la dénomination corps dissous. Cependant tous les corps dissous ne présentent pas cette affinité, par exemple les sucres, certains agents d'encollage type alcools polyvinyliques. Par ailleurs certains corps présentent une telle affinité chimique et ne sont pas dissous dans le fluide, ce sont par exemple les huiles sous forme liquide ou suspension. Ce procédé est caractérisé en ce que:

on immerge dans le fluide un élément qui comporte des centre capteurs capables de retenir chimiquement les corps dissous, ledit élément étant conformé de telle sorte que lesdits centres capteurs soient en contact avec le fluide en déplacement,

on laisse séjourner dans le fluide ledit élément pendant un intervalle de temps donné,

et on effectue l'analyse dudit élément afin de déceler la rétention éventuelle de corps dissous, présents dans le fluide pendant l'intervalle de temps donné, ce procédé ne comportant pas de filtration.

Ainsi le fluide lors de son déplacement vient au contact de la surface de l'élément qui a été immergé; les corps présents dans le fluide à l'état dissous sont retenus chimiquement par les centre capteurs que comporte l'élément, et ceci pendant tout le temps où l'élément est immergé. En conséquence lorsqu'on retire du fluide l'élément à la fin de l'intervalle de temps donné, les fonctions de rétention de l'élément ont retenu tous les corps dissous qui ont été présents dans le fluide, au contact de la surface de l'élément, pendant tout l'intervalle de temps où l'élément a été immergé, et l'analyse de l'élément permet de connaître avec précision quels ont été les corps dissous, présents dans le fluide pendant l'intervalle de temps. Dans les cas où on peut considérer que le fluide en déplacement est une solution homogène, c'est-à-dire où la concentration de tous les corps présents dans le fluide à l'état dissous à un moment donné est la même dans toute la section d'écoulement du fluide, alors il suffit que la surface de l'élément soit en contact avec une portion seulement du fluide; par contre si on considère que c'est une solution hétérogène, il est préférable que la surface de l'élément soit en contact avec la plus grande partie voire même tout le fluide en déplacement; cette précaution n'est bien sûr nécessaire que lorsqu'on désire connaître de manière exhaustive tous les corps dissous dans le fluide.

Avantageusement on immerge l'élément dans le fluide de manière à ce qu'une partie déterminée du fluide en déplacement vienne en contact avec ledit élément et on effectue une analyse quantitative de l'élément, moyennant quoi le procédé permet non seulement de détecter les corps dissous mais également de mesurer la concentration moyenne desdits corps dissous dans le fluide pendant l'intervalle de temps donné.

Si l'on a affaire à un fluide hétérogène, cette technique quantitative ne pourra être retenue que dans le cas où tout le fluide en déplacement vient en contact avec la surface de contact de l'élément; dans la plupart des cas cependant, il est possible de déterminer, grâce à la forme appropriée à l'élément, la portion de volume du fluide qui est venue en contact avec la surface de l'élément pendant l'intervalle de temps donné et, connaissant par l'analyse quantitative la quantité de corps dissous retenue grâce aux centre capteurs de l'élément, d'obtenir par le calcul la quantité totale de corps dissous qui ont été présents dans le fluide pendant l'intervalle de temps donné et donc leur concentration moyenne.

Que ce soit pour la détection uniquement ou pour le dosage des corps dissous, il importe que l'intervalle de temps soit déterminé de façon à ce que les fonctions de rétention de l'élément soient en nombre suffisant pour retenir tous les corps dissous dans le fluide venant en contact avec la surface de l'élément. En effet si les fonctions de rétention sont toutes saturées avant la fin de l'intervalle de temps, l'analyse de l'élément ne peut contenir que des indications imparfaites sur la teneur du fluide en corps dissous.

La surface de contact de l'élément avec le fluide en déplacement a une grande importance; en effet il importe que tout le fluide vienne en contact avec les centres capteurs de l'élément, pour que la détection puisse se faire efficacement. La notion de surface de contact doit être prise dans un sens large, il s'agit bien de la surface totale de l'élément qui peut venir en contact avec le fluide et non uniquement de la surface extérieure de l'élément; cette surface est donc considérablement augmentée lorsque le fluide peut pénétrer à l'intérieur de l'élément lui-même. Préférentiellement l'élément est réalisé dans une matière qui absorbe le fluide dans lequel il est immergé, par exemple une matière hydrophile lorsque le fluide est de l'eau; de la sorte le fluide pénètre dans la structure de l'élément et vient au contact des contres capteurs situés dans cette structure et non superficiellement. Malgré cela, lorsque la vitesse d'écoulement du fluide est faible, on observe que le fluide pénètre difficilement à l'intérieur de la structure si l'élément présente une certaine épaisseur; en effet dès que l'élément est immergé dans le fluide, il est totalement imprégné par le fluide est les corps dissous présents à cet instant dans le fluide sont retenus par les fonctions de rétention, mais par la suite le fluide ainsi imprégné reste lié à l'élément et au-delà d'une certaine épaisseur ne peut plus être déplacé par le fluide arrivant, il forme en quelque sorte une barrière qui s'oppose à la pénétration du fluide de renouvellement et donc à la rétention de nouveaux corps dissous. Aussi, préférentiellement, l'élément est conformé de telle sorte qu'il présente avec le fluide une très grande surface de contact sur une très faible épaisseur.

L'une des propriétés de l'élément est la capacité de retenir chimiquement les corps dissous dans le fluide grâce à des fonctions appropriées. En particulier les fonctions assurèes par les centre capteurs supportées par l'élément sont des fonctions échangeuses d'ions, susceptibles de retenir les corps dissous dans le fluide sous forme ionique; il peut également s'agir de fonctions retenant certains corps dissous par complexation ou par association chimique type oléophile ou organophile. La particularité des fonctions échangeuses d'ions est de retenir les ions correspondants sans distinction, tout au moins jusqu'à leur saturation: le corps dissous dans le fluide, à l'état ionique, déplace de la fonction échangeuse l'ion qui s'y trouvait initialement et se fixe sur cette fonction échangeuse. L'ion initial est choisi dans l'échelle d'affinité de manière à être déplacé par tous les corps que l'on souhaite retenir. En ce qui concerne la rétention par complexation, elle est plus spécifique de certains corps, et permet une rétention spécifique précise. En ce qui concerne la rétention par association oléophile, elle est spécifique des corps gras, elle permet en particulier la détection des huiles sous forme liquide ou suspension dans le fluide. Il peut s'agir plus généralement de toute fonction susceptible de retenir un corps dissous dans un fluide. Bien sûr le même élément peut comporter des fonctions de nature différente, suivant la nature des corps dissous que l'on souhaite détecter ou doser dans le fluide.

L'analyse de l'élément, pour déceler la présence de corps dissous, est fonction des corps recherchés. Si les corps en question sont des colorants, le simple examen visuel de l'élément permet le plus souvent de déterminer la présence ou l'absence de tels corps, et ceci même si l'examen visuel du flux qui s'écoule ne permet pas d'observer la présence de colorants, étant donné que les colorants, retenus sur l'élément, s'accumulent pendant tout l'intervalle de temps et sont de ce fait visibles. Dans le cas d'un dosage ou d'une détection de corps non colorés, il est nécessaire de régénérer les fonctions de rétention de l'élément, c'est-à-dire de déplacer par un traitement chimique approprié les corps retenus par d'autres corps, ce traitement de régénération permet d'obtenir une liqueur concentrée en corps dissous sur laquelle peut être réalisée la détection ou le dosage des corps de nouveau en solution, beaucoup plus facilement et précisément que s'il s'agit de solutions diluées. Par ailleurs, il n'est pas nécessaire d'effectuer le traitement de régénération sur l'élément dès que celui-ci est sorti du flux; l'élément peut être séché et stocké un certain temps avant que les fonctions ne soient régénérées et l'analyse effectuée.

C'est un autre objet de l'invention que de proposer un élément capteur spécialement conçu pour mettre en oeuvre le procédé précité. Cet élément capteur, destiné à être immergé dans le fluide en déplacement, est caractérisé en ce qu'il comporte des centre capteurs capables de retenir chimiquement les corps dissous présents dans le fluide et en ce qu'il est conformé de telle sorte que lesdits centres capteurs soient en contact avec le fluide en déplacement, cet élément capteur n'ayant pas de fonction de filtration. Avantageusement, l'élément capteur est réalisé dans une matière qui absorbe le fluide, de telle sorte que, le fluide pénétrant à l'intérieur de la structure de l'élément capteur, la surface de contact entre le fluide et l'élément en est grandement augmentée. Préférentiellement, lorsque la vitesse de fluide est faible, l'élément capteur a une faible épaisseur, ou est composé d'éléments unitaires qui ont eux-mêmes une faible épaisseur. En particulier il sera composé de fibres textiles, traitées de manière à ce qu'elles comportent des fonctions de rétention, et disposées sous forme de mèches, fils, bourres ou toute autre forme appropriée permettant un contact très étroit du fluide en déplacement avec chacune desdites fibres. Dans le cas où le fluide est une solution aqueuse, l'élément capteur sera avantageusement en matériau cellulosique, notamment en fils de coton ou de viscose.

Avantageusement les fonctions de rétention étant des fonctions échangeuses d'ions, de complexation ou d'association chimique, par exemple oléophile ou organophile, le matériau constitutif de l'élément capteur a été préalablement traité suivant les techniques de greffage de telle sorte qu'il comporte des chaînes greffées comprenant elles-mêmes des fonctions échangeuses d'ions,

de complexation ou d'association chimique, par exemple oléophile ou organophile. L'avantage procuré par la localisation desdites fonctions sur des chaînes greffées provient du fait que dans ce cas les chaînes gardent une relative mobilité par rapport au polymère de départ et que de ce fait les fonctions, concentrées sur ces chaînes, sont très accessibles au fluide et donc aux corps dissous dans le fluide.

L'élément capteur est immergé dans la canalisation dans laquelle se déplace le fluide. Il peut être placé directement dans le fluide ou être lui-même placé dans un dispositif adapté destiné à protéger l'élément capteur tout en n'étant pas un obstacle à la libre circulation du fluide en contact avec l'élément. Ce dispositif peut d'ailleurs être conçu en sorte de favoriser le contact entre le fluide et l'élément capteur, notamment en présentant des voies de passage obligé pour le fluide qui soient en contact avec l'élément capteur.

Pour le dosage des corps dissous, l'élément capteur, et éventuellement le dispositif qui le contient, est conformé de telle sorte que l'on peut déterminer la portion de fluide qui vient en contact avec lui par rapport à l'ensemble du fluide. Le cas le plus simple consiste bien sûr à dimensionner l'élément capteur de sorte que tout le fluide entre en contact avec lui lors de son déplacement. Mais le plus souvent, cette manière de faire entraîne des pertes de charge trop importantes qui rendent impossible sa mise en oeuvre, et l'élément capteur ou le dispositif n'entre en contact qu'avec une partie, voire même une faible partie du fluide total. Dans le cas de solution homogène, la connaissance de cette quote-part permet cependant, à partir de l'analyse quantitative des corps retenus sur l'élément pendant un intervalle de temps donné, de mesurer la quantité totale de corps ayant circulé dans le fluide et également la concentration moyenne du fluide en chacun de ces corps.

Les applications du procédé et de l'élément capteur selon l'invention sont multiples. Ils sont particulièrement bien adaptés à la détection de la pollution, par exemple dans les rivières: différents éléments capteurs placés le long du cours d'une rivière permettront de suivre l'évolution de la pollution et notamment de situer avec précision l'origine des effluents qui ont fait naître cette pollution. Ils permettent de détecter et de doser les produits toxiques, par exemple des produits radioactifs dans l'industrie nucléaire: pour cela l'élément capteur sera placé dans les conduits de circulation des bains contenant éventuellement de tels produits.

L'invention sera mieux comprise à l'aide des exemples décrits ci-après et illustrés par les dessins en annexe, dans lesquels:

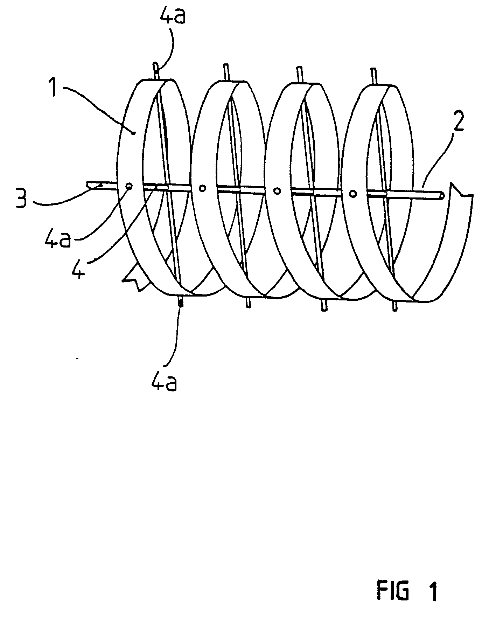

la figure 1 est une représentation schématique d'un élément capteur réalisé en matériau fibreux de cellulose greffée,

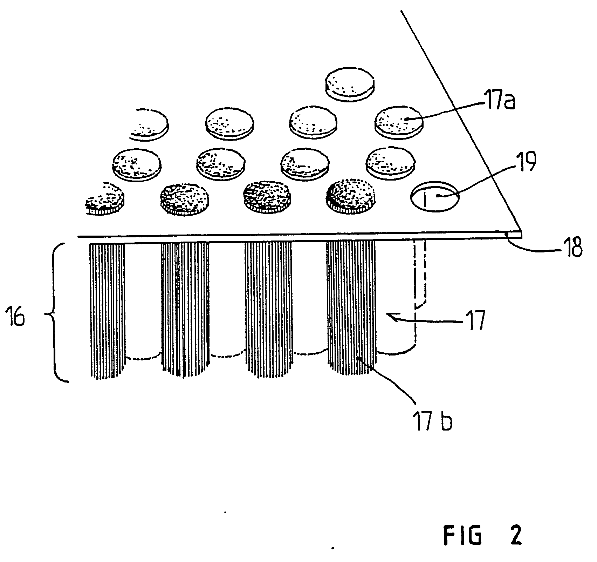

la figure 2 est une représentation schématique d'un élément capteur réalisé en fils de viscose greffée,

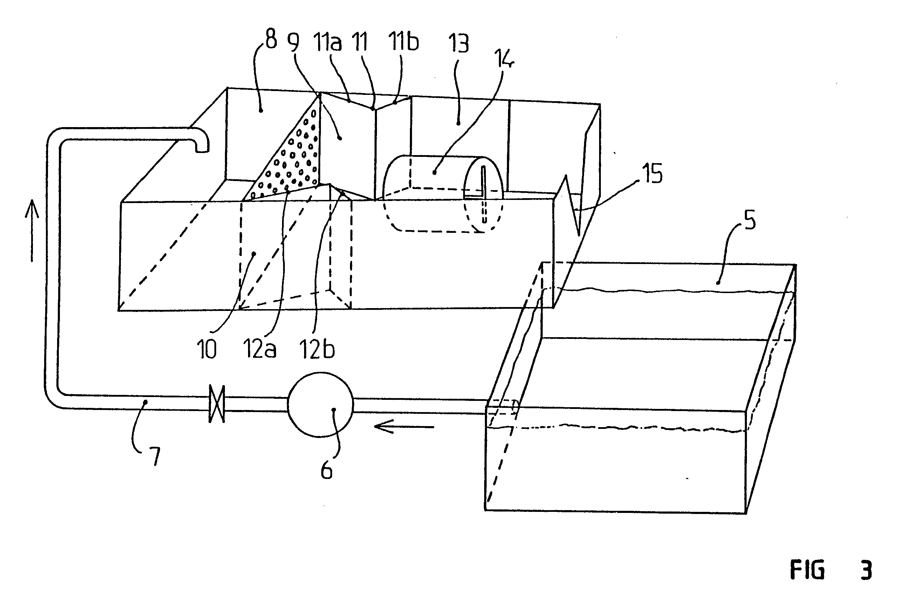

la figure 3 est une représentation schématique d'une installation de simulation dans laquelle est positionné un élément capteur.

Exemple 1

L'élément capteur 1 est réalisé en papier ou en non-tissé greffé. Le greffage consiste à développer sur un polymère de base différentes ramifications, appelées greffons, d'un autre polymère. Le greffage pratiqué sur le polymère cellulosique constitutif du papier ou du non-tissé est obtenu par imprégnation du polymère cellulosique à l'aide d'une solution aqueuse contenant 1% de soude et 15% en poids de chlorure époxypropyl triméthylammonium, essorage à 60%, séchage et thermofixation à 120°C pendant trois minutes, selon les enseignements du certificat d'addition français N° 72.20919. La cellulose ainsi greffée comporte des fonctions ammonium quaternaire qui sont des fonctions échangeuses d'anions, dont la forme ionique initiale est en $Cl^-$ ou $OH^-$.

La structure cellulosique est hydrophile, et possède une surface spécifique exceptionellement élevée notamment due à la structure fibreuse du papier ou du non-tissé: les fonctions échangeuses seront donc très accessibles aux ions dissous dans la solution. On a donné au papier ou non-tissé découpé en bandelette la forme d'une hélice; pour cela, la bandelette continue est placée sur un dispositif support 2 comprenant une tige centrale 3 sur laquelle sont fixées perpendiculairement à cette tige 3, des tiges secondaires 4; la bandelette 1 vient s'adapter sur les extrémités 4a des tiges 4.

Pour mettre en évidence le fonctionnement de l'invention, on a réalisé une installation simulant l'écoulement d'un liquide. Cette installation comporte un bac tampon 5, une conduite de transfert 7 du liquide du bac tampon 5 vers une première chambre 8, à l'aide de la pompe 6. Le liquide, injecté par la pompe 6, entre dans la chambre 8 et a obligatoirement un régime turbulent, ce qui favorise l'homogénéisation de la dissolution des corps dans le liquide. Le liquide passe ensuite dans une deuxième chambre 9 à travers une paroi perforée 10, son régime d'écoulement est soit turbulent pour les vitesses d'écoulement élevées, soit laminaire pour les vitesses faibles. Les parois latérales 11 et 12 de la chambre 9 sont constituées chacune de deux plaques (11a et 11b, 12a et 12b) qui sont réglables l'une par rapport à l'autre, pour former un angle plus ou moins prononcé. Ainsi en faisant varier le positionnement des différentes plaques, on peut obtenir des sections de passage du liquide différentes et donc des vitesses d'écoulement différentes. Le liquide passe enfin dans une troisième chambre 13 dans laquelle est placé le dispositif 14 supportant l'élément capteur. Après passage sur l'élément capteur le liquide se déverse par l'ouverture 15 dans le bac tampon 5 et peut être recyclé grâce à la pompe 6.

Le liquide mis en circulation dans l'installation est une solution aqueuse de fluorescéine à 0,5 mg/l; les essais à différentes vitesses montrent qu'à faible vitesse du liquide au niveau de l'élément capteur, de l'ordre de 0,1 m/s, la fixation de la fluorescéine est possible mais que le rende ment de fixation est faible, et que le rendement est meilleur à des vitesses plus élevées où le liquide peut pénétrer plus à coeur du papier ou non-tissé. L'analyse du papier ou du non-tissé pour la détection de la fluorescéine se fait soit visuellement: le papier est d'une couleur jaune a oranger en fonction de la quantité de fluorescéine retenue, soit à l'aide d'une lampe à ultraviolet.

Exemple 2

L'élément capteur 16 est réalisé en fils de viscose greffés comme décrit à l'exemple 1, disposés sous forme d'un ensemble de mèches 17, elles-mêmes étant fixées dans un support 18. A cet effet le support 18 est percé de trous 19 répartis en quinconce, et les têtes 17a des mèches 17 sont montées à force dans les trous 19, tandis que les queues 17b des mèches 17 dépassent largement du support 18. Ce support 18 peut être dans un matériau de densité plus légère que le fluide si l'on veut que le support 18 flotte à la surface du fluide et que les queues 17b des mèches soient totalement en contact avec le fluide en déplacement. Dans ce cas, on peut aisément choisir la longueur des mèches 17 en fonction de la profondeur de la canalisation dans laquelle on place l'élément capteur. La disposition en quinconce des mèches grâce à la disposition correspondante des trous 19 permet une bonne pénétration du fluide dans l'élément capteur même pour un fluide à faible vitesse de déplacement, à la manière de l'eau d'une rivière circulant à travers des algues.

L'élément capteur 16 et son dispositif support 18 sont placés dans la chambre 13 de l'installation décrite dans l'exemple 1. Le liquide mis en circulation est une solution aqueuse de chrome hexavalent à 1 mg/l sous forme de bichromate. Pour analyser la rétention de chrome sur les fonctions échangeuses d'anions des fils de viscose, on effectue sur les fils un traitement de régénération qui consiste à tremper les fils dans une solution de soude à concentration normale (Na OH 1N). Les ions bichromates sont déplacés et passent en solution sous forme de bichromate de sodium, tandis que la fonction échangeuse d'anions se retrouve sous forme $OH^-$. Les fils ainsi régénérés sont de nouveau prêtsà l'emploi, et il suffit d'analyser selon les méthodes classiques la solution sodée pour détecter ou quantifier la teneur en chrome.

La présentation en mèches est particulièrement intéressante lorsqu'on veut procéder à des analyses progressives dans le temps: il suffit de ne prélever du support 18 qu'une mèche 17, sur laquelle est faite l'analyse à un temps donné, et de laisser les autres mèches dans le fluide en vue d'analyses ultérieures. Par ailleurs la présentation n'est pas sensible au colmatage par des boues ou des matières en suspension dans le fluide, puisque les fils de viscose ne sont en aucun cas un obstacle au déplacement de ces matières dans le fluide.

Les exemples donnés ci-dessus ne sont en aucun cas limitatifs de l'invention. Toutes les fonctions de rétention connues de l'homme de l'art sont possibles; échangeuses d'anions ou de cations, selon par exemple l'enseignement du certificat d'addition du brevet français 72.25472, par complexation, oxydo-réduction etc... Sont possibles également toutes les formes de l'élément capteur, adaptées en fonction du fluide dans lequel il est placé, et éventuellement du moyen d'analyse envisagé, comme par exemple le comptage radioactif.

**Revendications**

1. Procédé pour detecter la présence dans un fluide en déplacement de certains corps notamment dissous comportant les operations suivantes:

on immerge dans le fluide un élément capteur (1, 16) comportant des centres capteurs capables de retenir chimiquement ces corps présents dans le fluide, ledit élément étant conformé de telle sorte que lesdits centre capteurs soient en contact avec le fluide en déplacement,

on laisse séjourner dans le fluide ledit élément (1, 16) pendant un intervalle de temps donné,

et on effectue l'analyse dudit élément (1, 16) afin de déceler la rétention éventuelle desdits corps, présents dans le fluide pendant 1' intervalle de temps donné, ce procédé ne comportant pas de filtration.

2. Procédé selon la revendication 1 caractérisé en ce qu'on immerge l'élément dans le fluide de manière à ce qu'une partie déterminée du fluide en déplacement vienne en contact avec ledit élément et on effectue une analyse quantitative de l'élément, moyennant quoi on peut ainsi mesurer la concentration moyenne des corps susceptibles de s'associer chimiquement avec les fonctions de l'élément capteur et présens dans le fluide pendant l'intervalle de temps donné.

3. Procédé selon l'une des revendications 1 et 2, caractérisé en ce que l'intervalle de temps est déterminé en sorte qu'il n'y ait pas saturation des fonctions de rétention de l'élément.

4. Procédé selon l'une des revendications 1, 2 et 3 caractérisé en ce que l'analyse est réalisée sur la solution de régénération des fonctions de rétention.

5. Elément capteur (1, 16) pour détecter la présence dans un fluide en déplacement de certains corps notamment dissous comportant des centre capteurs capables de retenir chimiquement lesdits corps et conformé de telle sorte que lesdits centres capteurs soient en contact avec le fluide en déplacement, cet élément capteur n'ayant pas de fonction de filtration.

6. Elément capteur (1, 16) selon la revendication 5 caractérisé en ce qu'il est réalisé dans une matière qui absorbe le fluide en déplacement.

7. Elément capteur (1, 16) selon la revendication 6 caractérisé en ce que, le fluide étant une solution aqueuse il est réalisé dans une matière hydrophile, en particulier cellulosique, notamment papier, coton ou viscose.

8. Elément capteur (1) selon la revendication 5 caractérisé en ce qu'il présente avec le fluide une très grande surface de contact sur une très faible épaisseur.

9. Elément capteur (16) selon la revendication 8 caractérisé en ce qu'il a la forme de fils, mèches ou bourre.

10. Elément capteur selon la revendication 9 caractérisé en ce qu'il est constitué d'un ensemble de mèches (17) dont les têtes (17a) sont fixées sur un support (18) et en ce que ledit support (18) a une densité plus faible que le fluide.

11. Elément capteur selon la revendication 5 caractérisé en ce qu'il comporte des centres capteurs pour des fonctions échangeuses d'anions, de cations, de complexation ou d'association chimique du type oléophile ou organophile, ou plusieurs de ces différentes fonctions.

12. Elément capteur selon la revendication 11 caractérisé en ce que ces centres capteurs sont portés par des chaînes greffées sur un polymère tronc.

13. Application du procédé selon l'une quelconque des revendications 1 à 4, à la détection de la pollution dans les fluides naturels.

14. Application du procédé selon l'une quelconque des revendications 1 à 4, à la détection de rejets radioactifs.

**Patentansprüche**

1. Verfahren zum Nachweis von gewissen, insbesondere gelösten Körpern in einem sich bewegenden Fluid mit folgenden Stufen:

Eintauchen in das Fluid eines Aufnahmeelements (1, 16) mit Aufnahmezentren, die die im Fluid vorhandenen Körper chemisch zurückhalten können, wobei das Element so ausgebildet ist, daß die Aufnahmezentren mit dem sich bewegenden Fluid in Kontakt sind.

Halten des Elements (1, 16) im Fluid während eines bestimmten Zeitintervalls und

Analyse des Elements (1, 16) zum Nachweis der eventuellen Retention der im Fluid während des Zeitintervalls vorhandenen Körper,
wobei das Verfahren keine Filtration umfaßt.

2. Verfahren nach Anspruch 1, dadurch gekennzeichnet, daß das Element in das Fluid so eingetaucht wird, daß ein bestimmter Teil des sich bewegenden Fluids mit dem Element in Kontakt kommt und daß eine quantitative Analyse des Elements durchgeführt wird, wodurch die mittlere Konzentration der Körper, die sich chemisch mit den Funktionen des Aufnahmeelements verbinden können und während des bestimmten Zeitintervalls im Fluid vorhanden sind, bestimmt werden kann.

3. Verfahren nach Anspruch 1 oder 2, dadurch gekennzeichnet, daß das Zeitintervall so bestimmt wird, daß keine Sättigung der Retentionsfunktionen des Elements auftritt.

4. Verfahren nach einem der Ansprüche 1—3, dadurch gekennzeichnet, daß die Analyse mit der Regenerationslösung der Retentionsfunktionen durchgeführt wird.

5. Aufnahmelement (1, 16) zum Nachweis von gewissen, insbesondere gelösten Körpern in einem sich bewegenden Fluid mit Aufnahmezentren, die diese Körper chemisch zurückhalten können, das so ausgebildet ist, daß die Aufnahmezentren mit dem sich bewegenden Fluid in Kontakt sind, wobei dieses Element keine Filtrierfunktion hat.

6. Aufnahmeelement (1, 16) nach Anspruch 5, dadurch gekennzeichnet, daß es aus einem das sich bewegende Fluid absorbierenden Material ist.

7. Aufnahmeelement (1, 16) nach Anspruch 6, dadurch gekennzeichnet, daß es aus einem hydrophilen Material, insbesondere cellulosehaltigen Material, wie Papier, Baumwolle oder Viskose ist, wenn das Fluid eine wäßrige Lösung ist.

8. Aufnahmeelement (1) nach Anspruch 5, dadurch gekennzeichnet, daß es eine sehr große Kontaktfläche mit dem Fluid bei sehr geringer Dicke hat.

9. Aufnahmeelement (16) nach Anspruch 8 in Form von Fäden, Dochten oder Flocken.

10. Aufnahmeelement nach Anspruch 9, dadurch gekennzeichnet, daß es aus einer Anordnung von Dochten (17) besteht, deren Köpfe (17a) auf einem Träger (18) befestigt sind, und daß der Träger (18) eine geringere Dichte als das Fluid hat.

11. Aufnahmeelement nach Anspruch 5, dadurch gekennzeichnet, daß es Aufnahmezentren für Anionen- oder Kationenaustauscherfunktionen, Komplexbildnerfunktionen oder chemische Bindungsfunktionen vom oleophilen oder organophilen Typ oder mehrere dieser verschiedenen Funktionen aufweist.

12. Aufnahmeelement nach Anspruch 11, dadurch gekennzeichnet, daß die Aufnahmezentren von Ketten getragen werden, die auf ein Polymerskelett aufgepfropft sind.

13. Anwendung des Verfahrens nach einem der Ansprüche 1—4 zum Nachweis der Verunreinigung von natürlichen Fluiden.

14. Anwendung des Verfahrens nach einem der Ansprüche 1—4 zum Nachweis von radioaktiven Abfällen.

**Claims**

1. Method for detecting the presence in a moving fluid of certain substances in particular dissolved ones, consisting in the following operations:

immersing in the fluid of a sensing element (1, 10) comprising sensing centers capable of chemically retaining such substances present in the fluid, said element being shaped in such a way that said sensing centers are in contact with the moving fluid,

leaving said element (1, 16) to dwell in the fluid for a given time period,

and making an analysis of said element (1, 16) in order to detect the possible retention of said substances present in the fluid during the given time period, said method comprising no filtering operation.

2. Method according to claim 1, characterized in that the element is immersed in the fluid in such a

way that a predetermined part of the moving fluid comes into contact with said element and in that a quantitative analysis of the element is made, whereby it is impossible to measure the mean concentration of any substances liable to become chemically associated with the functions of the sensing element and present in the fluid during the given time period.

3. Method according to one of claims 1 and 2, characterized in that the time period is determined so that there is no saturation of the element retention functions.

4. Method according to claims 1, 2 and 3, characterized in that the analysis is performed on the regenerating solution of the retention functions.

5. Sensing element (1, 16) for detecting the presence in a moving fluid of certain substances, in particular dissolved substances comprising sensing centers capable of chemically retaining said substances and so shaped that said sensing centers are in contact with the moving fluid, said sensing element having no filtering function.

6. Sensing element, according to claim 5, characterized in that it is produced in a material. which absorbs the moving fluid.

7. Sensing element (1, 16) according to claim 6, characterized in that the fluid being an aqueous solution, said element is produced in an hydrophilic and particularly cellulosic material such as paper, cotton or viscose.

8. Sensing element (1) according to claim 5, characterized in that it has a very large contacting surface with the fluid over a very small thickness.

9. Sensing element (16) according to claim 8, characterized in that it is in thread, rowe or block form.

10. Sensing element according to claim 9, characterized in that it is constituted of a bunch of rowes (17) of which the heads (17a) are fixed on a support (18) and in that said support (18) has a lower density than the fluid.

11. Sensing element according to claim 5, characterized in that it comprises sensing centers for detecting anion or cation exchanging functions, or oleophilic or organophilic type complexation or association, or a plurality of said functions.

12. Sensing element according to claim 11, characterized in that said sensing centers are carried by chains grafted on a trunk polymer.

13. Application of the method according to any one of claims 1 to 4, to detecting pollution in natural fluids.

14. Application of the method according to any one of claims 1 to 4, to detecting radioactive wastes.

0 159 230

FIG 1

FIG 2

FIG  3

8  9  11a  11  11b  13  14  15  5  10  12a  12b  7  6

0 159 230